(19) Europäisches Patentamt / European Patent Office / Office européen des brevets

(11) **EP 1 894 624 A1**

(12) **EUROPEAN PATENT APPLICATION**
published in accordance with Art. 153(4) EPC

(43) Date of publication:
**05.03.2008 Bulletin 2008/10**

(51) Int Cl.:
**B01J 23/58** (2006.01)   **B01D 53/94** (2006.01)
**F01N 3/10** (2006.01)

(21) Application number: **06756926.9**

(22) Date of filing: **02.06.2006**

(86) International application number:
**PCT/JP2006/311105**

(87) International publication number:
**WO 2006/134786 (21.12.2006 Gazette 2006/51)**

(84) Designated Contracting States:
**DE FR GB**

(30) Priority: **16.06.2005 JP 2005177044**

(71) Applicants:
• **Cataler Corporation**
 **Shizuoka-ken**
 **437-1492 (JP)**
• **DAIHATSU MOTOR COMPANY, LTD.**
 **Ikeda-shi,**
 **Osaka-fu 563-8651 (JP)**

(72) Inventors:
• **TANAKA, Hirohisa**
 **SHIGA TECHNICAL CENTER of**
 **Ryuo-cho, Gamo-gun, Shiga 5202593 (JP)**
• **TAN, Isao**
 **SHIGA TECHNICAL CENTER of DAIHATSU**
 **MOTOR**
 **Shiga 5202593 (JP)**
• **UENISHI, Mari**
 **SHIGA TECHNICAL CENTER of DAIHATSU**
 **Gamo-gun Shiga 5202593 (JP)**

• **TANIGUCHI, Masashi**
 **SHIGA TECHNICAL CENTER of**
 **Ryuo-cho Gamo-gun, Shiga 5202593 (JP)**
• **NAITO, Kazuya**
 **SHIGA TECHNICAL CENTER of DAIHATSU**
 **Gamo-gun, Shiga 5202593 (JP)**
• **KIMURA, Mareo**
 **c/o CATALER CORPORATION, 7800**
 **Shizuoka 4371492 (JP)**
• **NARITA, Keiichi**
 **c/o CATALER CORPORATION**
 **Shizuoka 4371492 (JP)**
• **SUZUKI, Hiromasa**
 **CATALER CORPORATION**
 **Shizuoka 4371492 (JP)**
• **MATSUEDA, Satoshi**
 **CATALER CORPORATION**
 **Kakegawa-shi, Shizuoka 4371492 (JP)**
• **ISHII, Yoshinori**
 **c/o CATALER CORPORATION**
 **Kakegawa-shi, Shizuoka 4371492 (JP)**

(74) Representative: **Schwabe - Sandmair - Marx**
 **Stuntzstrasse 16**
 **81677 München (DE)**

(54) **CATALYST COMPOSITIONS**

(57) To provide a catalyst composition containing a novel perovskite-type composite oxide which permits reversible entrance and exit of noble metals, a catalyst composition is prepared so as to contain a perovskite-type composite oxide represented by the general formula (1):

$$A_x A'_w B_y B'_{(1-y-z)} N_z O_{3\pm\sigma} \qquad (1)$$

(wherein A represents at least one element selected from alkaline earth metals; A' represents at least one element selected from rare earth elements; B represents a tetravalent rare earth element; B' represents at least one element selected from transition elements (excluding tetravalent rare earth elements, Rh, Pd and Pt); N represents at least one element selected from Rh, Pd and Pt; x and w represent an atomic ratio satisfying the following relations: $0.8 \leq x + w \leq 1.3$ ($0.8 < x \leq 1.3$, $0 \leq w \leq 0.4$); y represents an atomic ratio satisfying the following relation: $0 < y < 1.0$; z represents an atomic ratio satisfying the following relation: $0 < z \leq 0.5$; and $\sigma$ represents an oxygen excess or deficiency amount).

EP 1 894 624 A1

**Description**

Technical Field

The present invention relates to a catalyst composition which is used as a reaction catalyst for vapor or liquid phase.

Background Art

Noble metals such as Pt (platinum), Rh (rhodium) and Pd (palladium) have widely been used as catalytic active components of three-way catalysts which can simultaneously clean up carbon monoxide (CO), hydrocarbons (HC) and nitrogen oxides (NOx) contained in emissions.

It is also known that an exhaust gas purifying catalyst in which these noble metals are coordinated on a perovskite-type composite oxide having a crystal structure of the general formula: $ABO'_3$ shows high catalytic activity.

For example, it is reported that a perovskite-type composite oxide of $La_{1.00}Fe_{0.57}Co_{0.38}Pd_{0.05}O_3$ permits reversible entrance and exit of Pd to and from a perovskite-type crystal structure corresponding to oxidation-reduction change of emissions, and suppresses grain growth and maintains high catalytic activity over a long time because of such a self-regenerative function (see Non-Patent Document 1).

Examples of the perovskite-type composite oxide in which the noble metal is coordinated include $La_{0.90}Sr_{0.10}Fe_{0.54}Mn_{0.36}Pt_{0.10}O_3$, $La_{0.95}Ag_{0.05}Al_{0.80}Mn_{0.10}Pt_{0.08}Ru_{0.02}O_3$, $La_{0.9}Ce_{0.1}Co_{0.9}Pt_{0.05}Ru_{0.05}O_3$, $Nd_{0.80}Ba_{0.10}Mg_{0.10}Al_{0.85}Pt_{0.10}Rh_{0.05}O_3$, $La_{0.90}Ca_{0.10}Fe_{0.90}Pt_{0.10}O_3$, $Y_{0.50}Sr_{0.50}Fe_{0.50}Pt_{0.50}O_3$, $La_{0.90}Sr_{0.10}Mn_{0.90}Pt_{0.10}O_3$ (see, for example, Patent Document 1), $La_{1.00}Fe_{0.95}Rh_{0.05}O_3$, $La_{0.70}Pr_{0.30}Fe_{0.95}Rh_{0.05}O_3$, $La_{0.80}-Nd_{0.15}-Ce_{0.05}Fe_{0.60}Mn_{0.35}Rh_{0.05}O_3$, $La_{0.90}Y_{0.10}Fe_{0.70}Al_{0.20}Rh_{0.10}O_3$, Pt-supported/$La_{1.00}Al_{0.95}Rh_{0.05}O_3$, $La_{0.80}-Ce_{0.20}-Fe_{0.65}Rh_{0.35}O_3$, $La_{1.00}Fe_{0.90}Rh_{0.105}O_3$, $La_{1.00}Fe_{0.60}Mn_{0.30}Rh_{0.10}O_3$ (see, for example, Patent Document 2), $La_{1.00}Fe_{0.95}Pd_{0.05}O_3$, $La_{1.00}Fe_{0.90}Pd_{0.10}O_3$, $La_{1.00}Fe_{0.57}Mn_{0.38}Pd_{0.05}O_3$, and $La_{0.80}Nd_{0.20}Fe_{0.90}Pd_{0.10}O_3$ (see, for example, Patent Document 3).

Non-Patent Document 1: Y. Nishihata et al., Nature, Vol. 418, No. 6894, pp. 164-167, 11 July, 2002
Patent Document 1: Japanese Unexamined Patent Publication No. 2004-041868
Patent Document 2: Japanese Unexamined Patent Publication No. 2004-041867
Patent Document 3: Japanese Unexamined Patent Publication No. 2004-041866

Disclosure of the Invention

Problems to be Solved by the Invention

However, in the above perovskite-type composite oxide, since it is necessary to coordinate the noble metal so as to reversibly enter and exit in the crystal structure, specific elements must be combined to form a crystal structure. Therefore, raw materials to be used are limited so as to prepare the above perovskite-type composite oxide, and it may become difficult to stably prepare the perovskite-type composite oxide industrially because of variation in price of raw materials or other problems. Thus, it is desired to propose a novel crystal structure which can permit reversible entrance and exit of noble metals.

An object of the present invention is to provide a catalyst composition containing a novel perovskite-type composite oxide which can permit reversible entrance and exit of noble metals.

Solution to the Problem

To achieve the above object, the catalyst composition of the present invention includes a perovskite-type composite oxide represented by the general formula (1) :

$$A_xA'_wB_yB'_{(1-y-z)}N_zO_{3\pm\sigma} \qquad (1)$$

(wherein A represents at least one element selected from alkaline earth metals; A' represents at least one element selected from rare earth elements; B represents a tetravalent rare earth element; B' represents at least one element selected from transition elements (excluding tetravalent rare earth elements, Rh, Pd and Pt); N represents at least one element selected from Rh, Pd and Pt; x and w represent an atomic ratio satisfying the following relations: $0.8 \le x + w \le$

1.3 ($0.8 \leq x \leq 1.3$, $0 \leq w \leq 0.4$); y represents an atomic ratio satisfying the following relation: $0 < y < 1.0$; z represents an atomic ratio satisfying the following relation: $0 < z \leq 0.5$; and σ represents an oxygen excess or deficiency amount).

In the present invention, B preferably represents at least one element selected from Ce, Pr and Tb in the general formula (1).

In the present invention, N preferably represents Pt in the general formula (1).

In the present invention, at least one element selected from Rh, Pd and Pt and represented by N in the general formula (1) preferably exists as a solid solution at an amount of 50% by weight based on each content of at least one element selected from Rh, Pd and Pt and represented by N in the general formula (1) in a perovskite-type composite oxide in an oxidative atmosphere.

The catalyst composition of the present invention is preferably an exhaust gas purifying catalyst.

Effect of the Invention

In the catalyst composition containing the perovskite-type composite oxide of the present invention, since solid solution-precipitation (self-regeneration) in which the noble metal is transformed into a solid solution in the perovskite-type composite under an oxidative atmosphere and precipitated from the same under a reducing atmosphere are efficiently repeated, a dispersion state of them can be maintained, and thus deterioration in activity due to grain growth can be prevented and high catalytic activity can be maintained over a long time. Therefore, the catalyst composition can be widely used as a reaction catalyst for gas or liquid phase.

Embodiment of the Invention

The catalyst composition of the present invention contains a perovskite-type composite oxide represented by the general formula (1):

$$A_x A'_w B_y B'_{(1-y-z)} N_z O_{3 \pm \sigma} \qquad (1)$$

(wherein A represents at least one element selected from alkaline earth metals; A' represents at least one element selected from rare earth elements; B represents a tetravalent rare earth element; B' represents at least one element selected from transition elements (excluding tetravalent rare earth elements, Rh, Pd and Pt); N represents at least one element selected from Rh, Pd and Pt; x and w represent an atomic ratio satisfying the following relations: $0.8 \leq x + w \leq 1.3$ ($0.8 \leq x \leq 1.3$, $0 \leq w \leq 0.4$); y represents an atomic ratio satisfying the following relation: $0 < y < 1.0$; z represents an atomic ratio satisfying the following relation: $0 < z \leq 0.5$; and σ represents an oxygen excess or deficiency amount).

That is, the composite oxide has a perovskite-type structure, and at least one element selected from alkaline earth metals is coordinated as A and at least one element selected from rare earth metals is coordinated as A' on the site A. On the site B, a tetravalent rare earth element is coordinated as B and, if necessary, at least one element selected from transition elements excluding tetravalent rare earth elements, Rh, Pd and Pt are coordinated as B' on the B site, and furthermore, at least one selected from Rh, Pd and Pt is coordinated as N on the site B.

In the general formula (1), examples of the alkaline earth metals represented by A include Be (beryllium), Mg (magnesium), Ca (calcium), Sr (strontium), Ba (barium) and Ra (radium), of which Ca, Sr and Ba are preferred. These alkaline earth metals can be used alone or in combination.

In general formula (1), examples of the rare earth elements represented by A' include rare earth elements whose valence does not vary from 3, such as Sc (scandium), Y (yttrium), La (lanthanum), Nd (neodymium), Pm (promethium), Gd (gadolinium), Dy (dysprosium), Ho (holmium), Er (erbium) and Lu (lutetium); rare earth elements whose valence varies to 3 or 4, such as Ce (cerium), Pr (praseodymium) and Tb (terbium); and rare earth elements whose valence varies to 2 or 3, such as Sm (samarium), Eu (europium), Tm (thulium) and Yb (ytterbium). These rare earth elements can be used alone or in combination.

In the general formula (1), on the site A, x and w represent an atomic ratio satisfying the following relations: $0.8 \leq x + w \leq 1.3$ ($0.8 \leq x \leq 1.3$, $0 \leq w \leq 0.4$), and preferably $0.9 \leq x + w \leq 1.3$ ($0.9 \leq x \leq 1.3$, $0 \leq w \leq 0.4$), namely, the total atomic ratio (w + x) of the elements (A and A') coordinated on the site A is 0.8 or more and 1.3 or less, and preferably 0.9 or more and 1.3 or less. When (w + x) is 0.8 or more, Rh, Pd and/or Pt can be stably transformed into a solid solution in a higher rate of a solid solution. When (w + x) exceeds 1.3, by-products other than the above composite oxides may be produced.

Further, x represents an atomic ratio satisfying the following relation: $0.8 \leq x \leq 1.3$, and preferably $0.9 \leq x \leq 1.3$. Namely, the alkaline earth metals represented by A are certainly contained in the atomic ratio of 0.8 or more and 1.3 or less, and preferably 0.9 or more and 1.3 or less.

On the other hand, w represents an atomic ratio satisfying the following relation: $0 \leq w \leq 0.4$. Namely, the rare earth metals represented by A' are not contained (w = 0) when the atomic ratio of the alkaline earth elements represented by A is 1.3 (x = 1.3). When the atomic ratio of the alkaline earth elements is 0.8 or more and less than 1.3 ($0.8 \leq x < 1.3$), the rare earth metals are optionally contained in the atomic ratio of 0.4 or less ($0 \leq w \leq 0.4$).

Examples of the tetravalent rare earth element represented by B on the site B include Ce (cerium), Pr (praseodymium) and Tb (terbium), of which Ce is preferred.

The transition elements excluding tetravalent rare earth elements, Rh, Pd and Pt, which are represented by B' on the site B, are not specifically limited and include elements having atomic numbers of 21 (Sc) through 30 (Zn), atomic numbers of 39 (Y) through 48 (Cd), and atomic numbers of 57 (La) through 80 (Hg) in the Periodic Table of Elements (IUPAC, 1990), but exclude tetravalent rare earth elements, Rh, Pd and Pt. Specific examples thereof include Zr (zirconium), Ti (titanium), Y (yttrium) and Nd (neodymium).

These transition elements may be used alone or in combination.

On the site B, Rh (rhodium), Pd (palladium) and Pt (platinum) represented by N may be used alone or in combination. Among these elements, Pt is preferred.

In the general formula (1), on the site B, y represents an atomic ratio satisfying the following relation: $0 < y < 1.0$, and preferably $0.4 < y < 1.0$, and z represents an atomic ratio satisfying the following relation: $0 < z \leq 0.5$, and preferably $0 < z < 0.1$. Namely, on the site B, a tetravalent rare earth element represented by B is certainly coordinated at an atomic ratio of less than 1 and at least one element represented by N selected from Rh, Pd and Pt is certainly coordinated at an atomic ratio of 0.5 or less. Further, at least one element represented by B' selected from transition elements excluding tetravalent rare earth elements, Rh, Pd and Pt is optionally coordinated at an atomic ratio of (1-y-z), namely, a residual atomic ratio from tetravalent rare earth elements represented by B and at least one element represented by N selected from Rh, Pd and Pt on the site B. When the total atomic ratio of tetravalent rare earth elements represented by B and at least one element represented by N selected from Rh, Pd and Pt is 1 on the site B, at least one element represented by B' selected from transition elements excluding tetravalent rare earth elements, Rh, Pd and Pt is not coordinated.

In the general formula (1), σ represents an oxygen excess or deficiency amount, which shows that most has an $ABO_3$ type perovskite-type structure.

The composite oxide of the present invention having a perovskite structure can be prepared according to any suitable procedure for the preparation of composite oxides, such as coprecipitation method, citrate complex method and alkoxide method, without specific limitations.

In the coprecipitation method, for example, an aqueous mixed salt solution containing salts of the above-mentioned respective elements in the above-mentioned stoichiometric ratio is prepared. The aqueous mixed salt solution is coprecipitated by addition of a neutralizing agent, and the resulting coprecipitate is dried and subjected to a heat treatment.

Examples of the salts of the respective elements are inorganic salts such as sulfates, nitrates, chlorides and phosphates; and organic salts such as acetates and oxalates. The aqueous mixed salt solution can be prepared, for example, by adding the salts of the respective elements to water so as to establish the above-mentioned stoichiometric ratio and mixing them with stirring.

Then, the aqueous mixed salt solution is coprecipitated by adding the neutralizing agent thereto. Examples of the neutralizing agent include, but are not specifically limited to, ammonia; organic bases including amines such as triethylamine and pyridine; and inorganic bases such as sodium hydroxide, potassium hydroxide, potassium carbonate and ammonium carbonate. The neutralizing agent is added dropwise so that the solution after the addition of the neutralizing agent has a pH of about 6 to 10. This dropwise addition efficiently coprecipitates the salts of the respective elements.

The resulting coprecipitate is washed with water, if necessary, dried by vacuum drying or forced-air drying, for example, and subjected to a heat treatment, for example, at about 500 to 1,200°C, preferably at about 600 to 1,000°C. Thus, a perovskite-type composite oxide can be prepared.

In the citrate complex method, for example, an aqueous citric acid mixed salt solution is prepared by adding a aqueous citric acid to a aqueous mixed salt solution so that an amount of the citric acid slightly exceeds an amount corresponding to the stoichiometric ratio with respect to the above-mentioned respective elements. The aqueous citric acid mixed salt solution is evaporated to dryness to form citrate complex of the respective elements. The resulting citrate complex is provisionally baked and then subjected to a heat treatment.

The evaporation to dryness is carried out to remove fluid at a temperature at which the formed citrate complex is not decomposed, for example, at room temperature to about 150°C.

The provisional baking may be carried out by heating at a temperature of 250 to 350°C in vacuum or in an inert atmosphere. The provisionally baked citrate complex is then heated, for example, at about 500 to 1,200°C, and preferably at about 600 to 1,000°C to obtain a perovskite-type composite oxide.

In the alkoxide method, for example, an alkoxide mixed solution containing alkoxides of the respective elements, excluding Rh, Pd and Pt, in the above-mentioned stoichiometric ratio is prepared. The alkoxide mixed solution is precipitated on hydrolysis to form a precipitate. Then a slurry is prepared and added with an aqueous solution containing salts of the noble metals of Rh, Pd and Pt thereto. The resulting precipitate is dried and then subjected to a heat treatment.

Examples of the alkoxides of the respective elements include alcholates each comprising the respective element and an alkoxy such as methoxy, ethoxy, propoxy, isopropoxy or butoxy; and alkoxyalcholates of the respective elements represented by the following general formula (2):

$$E[OCH(R^1)-(CH_2)_i-OR^2]_j \qquad (2)$$

(wherein E represents the respective element; $R^1$ represents a hydrogen atom or an alkyl group having 1 to 4 carbon atoms; $R^2$ represents an alkyl group having 1 to 4 carbon atoms; i is an integer of 1 to 3; and j is an integer of 2 to 4).

More specific examples of the alkoxyalcholates include methoxyethylate, methoxypropylate, methoxybutylate, ethoxyethylate, ethoxypropylate, propoxyethylate and butoxyethylate.

The alkoxide mixed solution can be prepared, for example, by adding the alkoxides of the respective elements to an organic solvent in such proportions so as to establish the above-mentioned stoichiometric ratio and mixing them with stirring. The organic solvent is not specifically limited, as long as it can dissolve the alkoxides of the respective elements. Examples of such organic solvents include aromatic hydrocarbons, aliphatic hydrocarbons, alcohols, ketones and esters. Among them, aromatic hydrocarbons such as benzene, toluene and xylenes are preferred.

Then, the alkoxide mixed solution is precipitated on hydrolysis by adding water to form a precipitate. The organic solvent is distilled off from the alkoxide mixed solution to prepare a slurry, and an aqueous solution containing salts of Rh, Pd and Pt hereto is added to the slurry at a predetermined stoichiometric ratio.

Examples of the aqueous solution containing salts of Rh, Pd and Pt include aqueous nitrate solution, aqueous chloride solution, aqueous hexaammine chloride solution, aqueous dinitrodiammine nitrate solution, hexachloro acid hydrate and potassium cyanide salt.

The resulting precipitate is dried, for example, by vacuum drying or forced-air drying to distill off water and is subjected to a heat treatment (baking), for example, at about 500 to 1,200°C, and preferably at about 600 to 1,000°C. Thus, a perovskite-type composite oxide can be prepared.

In the alkoxide method, for example, the composite oxide may be alternatively prepared in the following manner. A solution containing organometallic salts of the noble metals of Rh, Pd and Pt is added to the above-mentioned alkoxide mixed solution to obtain a homogenous mixed solution. The homogenous mixed solution is precipitated on hydrolysis by adding water thereto. The resulting precipitate is dried and then subjected to a heat treatment.

Examples of the organometallic salts of Rh, Pd and Pt include carboxylates of the above-mentioned noble metals derived from, for example, acetates and propionates; metal chelate complexes of the above-mentioned noble metals derived from, for example, β-diketone compounds or β-ketoester compounds represented by the following general formula (3) and/or β-dicarboxylic ester compounds represented by the following general formula (4):

$$R^3COCHR^5COR^4 \qquad (3)$$

(wherein $R^3$ represents an alkyl group having 1 to 6 carbon atoms, a fluoroalkyl group having 1 to 6 carbon atoms or an aryl group; $R^4$ represents an alkyl group having 1 to 6 carbon atoms, a fluoroalkyl group having 1 to 6 carbon atoms, an aryl group, or an alkyloxy group having 1 to 4 carbon atoms; and $R^5$ represents a hydrogen atom or an alkyl group having 1 to 4 carbon atoms); and

$$R^7CH(COOR^6)_2 \qquad (4)$$

(wherein $R^6$ represents an alkyl group having 1 to 6 carbon atoms, and $R^7$ represents a hydrogen atom or an alkyl group having 1 to 4 carbon atoms).

In above general formulas (3) and (4), examples of the alkyl group having 1 to 6 carbon atoms of $R^3$, $R^4$ and $R^6$ include methyl, ethyl, propyl, isopropyl, n-butyl, s-butyl, t-butyl, t-amyl and t-hexyl. Examples of the alkyl group having 1 to 4 carbon atoms of $R^5$ and $R^7$ include methyl, ethyl, propyl, isopropyl, n-butyl, s-butyl and t-butyl. Examples of the fluoroalkyl group having 1 to 6 carbon atoms of $R^3$ and $R^4$ include trifluoromethyl. Examples of the aryl group of $R^3$ and $R^4$ include phenyl. Examples of the alkyloxy group having 1 to 4 carbon atoms of $R^4$ include methoxy, ethoxy, propoxy, isopropoxy, n-butoxy, s-butoxy and t-butoxy.

Specific examples of the β-diketone compound include 2,4-pentanedione, 2,4-hexanedione, 2,2-dimethyl-3,5-hexanedione, 1-phenyl-1,3-butanedione, 1-trifluoromethyl-1,3-butanedione, hexafluoroacetylacetone, 1,3-diphenyl-1,3-propanedione and dipivaloylmethane.

Specific examples of the β-ketoester compound include methyl acetoacetate, ethyl acetoacetate and t-butyl acetoacetate.

Specific examples of the β-dicarboxylic ester compound include dimethyl malonate and diethyl malonate.

The solution containing the organometallic salts of Rh, Pd and Pt can be prepared, for example, by adding an organometallic salt of Rh, Pd and Pt to an organic solvent in such proportions as to establish the above-mentioned stoichiometric ratio, and mixing them with stirring. The organic solvent can be any of the above organic solvents.

The thus prepared solution containing the organometallic salts of Rh, Pd and Pt is added to the alkoxide mixed solution to obtain a homogenous mixed solution, and the homogenous mixed solution is precipitated on hydrolysis by adding water thereto. The resulting precipitate is dried, for example, by vacuum drying or forced-air drying and is subjected to a heat treatment, for example, at about 400 to 1,000°C, and preferably at about 500 to 850°C. A perovskite-type composite oxide is thus prepared.

Alternatively, the perovskite-type composite oxide of the present invention can be obtained by preparing a perovskite-type composite oxide from the above respective elements excluding Rh, Pd and Pt in the above-mentioned stoichiometric ratio using the above coprecipitation method, citrate complex method or alkoxide method, and transforming Rh, Pd and Pt into a solid solution in the resulting perovskite-type composite oxide in the above-mentioned stoichiometric ratio to form a solid solution.

A method of transforming Rh, Pd and Pt into a solid solution in the perovskite-type composite oxide to form a solid solution is not specifically limited and a known method can be used. For example, a solution of salts containing Rh, Pd and Pt is prepared and the perovskite-type composite oxide is impregnated with the salt containing solution and then baked.

As the salt containing solution, a solution of the above-illustrated salt may be used and, practically aqueous nitrate solution, dinitrodiammine nitrate solution and aqueous chloride solution are used.

Specific examples of a palladium salt solution include aqueous palladium nitrate solution, dinitrodiammine palladium nitrate solution and palladium tetraammine nitrate solution; specific examples of a rhodium salt solution include rhodium nitrate solution and rhodium chloride solution; and specific examples of a platinum salt solution include dinitrodiammine platinum nitrate solution, chloroplatinic acid solution and platinum tetraammine solution. The perovskite-type composite oxide is impregnated with the noble metal, and then dried, for example, at 50 to 200°C for about 1 to 48 hours and further baked at about 350 to 1,000°C for 1 to 12 hours.

The thus prepared perovskite-type composite oxide according to the present invention can be used intact as an exhaust gas purifying catalyst but is generally prepared as a catalyst composition by a known method such as being supported on a catalyst carrier.

The catalyst carrier is not specifically limited and includes, for example, known catalyst carriers such as honeycomb monolith carriers made of cordierite and the like.

The perovskite-type composite oxide may be supported on the catalyst carrier, for example, by adding water to the perovskite-type composite oxide to form a slurry, applying the slurry to the catalyst carrier, drying the applied slurry and subjecting it to heat treatment at about 300 to 800°C, and preferably at about 300 to 600°C.

In the thus obtained catalyst composition containing the perovskite-type composite oxide of the present invention, Rh, Pd and Pt transform at a high rate into a solid solution in a crystal structure of the perovskite-type composite oxide, and the transformed Rh, Pd and Pt are precipitated from the crystal structure under a reducing atmosphere, or transformed into a solid solution in the crystal structure under an oxidative atmosphere. A rate of a solid solution of Rh, Pd and Pt in the crystal structure of the perovskite-type composite oxide can be adjusted by a final baking temperature and a baking time.

In the present invention, at least one element represented by N selected from Rh, Pd and Pt in the general formula (1) exists as a solid solution preferably at an amount of 50% or more by weight, and more preferably from 80 to 1.00% by weight in a perovskite-type composite oxide under an oxidative atmosphere, based on each content ratio of at least one element represented by N selected from Rh, Pd and Pt in the general formula (1). When the proportion (a rate of solid solution) of at least one element selected from Rh, Pd and Pt as a solid solution is less than 50% by weight, deterioration in activity of a catalyst composition as a catalyst may be caused by sintering of the at least one element selected from Rh, Pd and Pt.

A rate of a solid solution of the noble metals can be calculated, for example, using a measuring method in Examples described hereinafter.

Thus, in the catalyst composition of the present invention, by a self-regenerative function capable of repeating formation of solid solution under an oxidative atmosphere and precipitation under a reducing atmosphere, grain growth of Rh, Pd and Pt is effectively suppressed and a dispersion state of them can be maintained even in longterm use. As a result, high catalytic activity can be maintained over a long time even if the used amount of Rh, Pd and Pt is remarkably decreased.

In the catalyst composition of the present invention, when the atomic proportion of the element coordinated on the site A exceeds 1, a rate of a solid solution of Rh, Pd and Pt can be more increased and the catalyst composition can be provided with stable quality.

Therefore, the catalyst composition containing the perovskite-type composite oxide of the present invention can be widely used as a reaction catalyst for gas or liquid phase. In particular, the catalyst composition can realize excellent exhaust gas purifying properties over a long time, and therefore, the catalyst composition can be used an exhaust gas purifying catalyst which is used for purifying exhaust gas exhausted from internal combustion engines such as gasoline engine and diesel engine, and boilers.

EXAMPLES

The present invention will be illustrated in further detail with reference to several examples and comparative examples below, which are never intended to limit the scope of the invention.

Example 1

(Production of $Ba_{1.00}Ce_{0.93}P_{0.07}O_3$)
Barium methoxypropylate     (Ba content: 0.100 mol)
Cerium methoxypropylate     (Ce content: 0.093 mol)

An alkoxide mixed solution was prepared by charging the above components in a round-bottomed flask and dissolving them in toluene added thereto with stirring. Then, platinum acetylacetonate (Pt content: 0.007 mol) was dissolved in toluene to prepare an organometallic salt solution, and the resulting solution was added to the alkoxide mixed solution in the round-bottomed flask to obtain a homogenous mixed solution containing BaCePt.

Next, deionized water was added dropwise in the round-bottomed flask over about 15 minutes to form a viscous precipitate on hydrolysis.

After stirring at room temperature for 2 hours, toluene and water were distilled off under reduced pressure to obtain a precursor of the BaCePt composite oxide. The precursor was subjected to forced-air drying at 60°C for 24 hours and subjected to a heat treatment (baking) at 650°C in the atmosphere for 2 hours using an electric furnace to obtain a powder of a perovskite-type composite oxide (Pt content: 4.15% by weight) having a structure of $Ba_{1.00}Ce_{0.93}Pt_{0.07}O_3$.

The X-ray powder diffraction analysis of the powder revealed that it was identified as a single crystal phase comprising a Pt-containing perovskite-type composite oxide having a structure of $Ba_{1.00}Ce_{0.93}Pt_{0.07}O_3$.

Example 2

(Production of $Ba_{0.95}Ce_{0.465}Pr_{0.465}Pt_{0.07}O_{3-\sigma}$)
Barium isopropoxide          (Ba content: 0.095 mol)
Cerium isopropoxide          (Ce content: 0.0465 mol)
Praseodymium isopropoxide    (Pr content: 0.0465 mol)

An alkoxide mixed solution was prepared by charging the above components in a round-bottomed flask and dissolving them in toluene added thereto with stirring. Deionized water was added to the alkoxide mixed solution to form a viscous white precipitate on hydrolysis. Toluene was distilled off from the alkoxide mixed solution to obtain a slurry, and an aqueous dinitrodiammine platinum nitrate solution (Pt content: 0.007 mol) was added to the slurry, followed by stirring at room temperature for one hour.

Water was distilled off to dryness under reduced pressure to obtain a precursor of a BaCePrPt composite oxide. The precursor was subjected to a heat treatment (baking) at 800°C in the atmosphere for 2 hours using an electric furnace to obtain a powder of a Pt-containing perovskite-type composite oxide (Pt content: 4.23% by weight) having a structure of $Ba_{0.95}Ge_{0.465}Pr_{0.465}Pt_{0.07}O_{3-\sigma}$. The X-ray powder diffraction analysis of the powder revealed that it was identified as a single crystal phase comprising a pt-containing perovskite-type composite oxide having a structure of $Ba_{0.95}Ce_{0.465}Pr_{0.465}Pt_{0.07}O_{3-\sigma}$.

Example 3

(Production of $Ca_{0.80}Ce_{0.85}Pt_{0.05}O_{3-\sigma}$)
Calcium isopropoxide     (Ca content: 0.080 mol)
Cerium isopropoxide      (Ce content: 0.095 mol)

The above components were charged in a round-bottomed flask and, after treating in the same manner as in Example 2, an aqueous dinitrodiammine platinum nitrate solution (Pt content: 0.005 mol) was added, followed by stirring at room temperature for one hour.

Next, water was distilled off to dryness under reduced pressure to obtain a precursor of a CaCePt composite oxide. The precursor was subjected to a heat treatment (baking) at 800°C in the atmosphere for 2 hours using an electric furnace to obtain a powder of a Pt-containing perovskite-type composite oxide (Pt content: 4.38% by weight) having a structure of $Ca_{0.80}Ce_{0.95}Pt_{0.05}O_{3-\sigma}$.

The X-ray powder diffraction analysis of the powder revealed that it was identified as a single crystal phase comprising a Pt-containing perovskite-type composite oxide having a structure of $Ca_{0.80}Ce_{0.95}Pt_{0.05}O_{3-\sigma}$.

Example 4

(Production of $Ca_{1.02}Ce_{0.475}Zr_{0.475}Pt_{0.05}O_{3+\sigma}$)
Calcium isopropoxide     (Ca content: 0.102 mol)
Cerium isopropoxide     (Ce content: 0.475 mol)
Zirconium isopropoxide     (Zr content: 0.0475 mol)

The above components were charged in a round-bottomed flask and, after treating in the same manner as in Example 2, an aqueous dinitrodiammine platinum nitrate solution (Pt content: 0.005 mol) was added, followed by stirring at room temperature for one hour.

Next, water was distilled off to dryness under reduced pressure to obtain a precursor of a CaCeZrPt composite oxide. The precursor was subjected to a heat treatment (baking) at 800°C in the atmosphere for 2 hours using an electric furnace to obtain a powder of a Pt-containing perovskite-type composite oxide (Pt content: 4.68% by weight) having a structure of $Ca_{1.02}Ce_{0.475}Zr_{0.475}Pt_{0.05}O_{3+\sigma}$.

The X-ray powder diffraction analysis of the powder revealed that it was identified as a single crystal phase comprising a Pt-containing perovskite-type composite oxide having a structure of $Ca_{1.02}C_{0.475}Zr_{0.475}Pt_{0.05}O_{3+\sigma}$

Example 5

(Production of $Sr_{0.90}Ce_{0.94}Pt_{0.06}O_{3-\sigma}$)
Strontium isopropoxide     (Sr content: 0.090 mol)
Cerium isopropoxide     (Ce content: 0.094 mol)

The above components were charged in a round-bottomed flask and, after treating in the same manner as in Example 2, an aqueous dinitrodiammine platinum nitrate solution (Pt content: 0.006 mol) was added, followed by stirring at room temperature for one hour.

Next, water was distilled off to dryness under reduced pressure to obtain a precursor of a SrCePt composite oxide. The precursor was subjected to a heat treatment (baking) at 800°C in the atmosphere for 2 hours using an electric furnace to obtain a powder of a Pt-containing perovskite-type composite oxide (Pt content: 4.33% by weight) having a structure of $Sr_{0.90}Ce_{0.94}Pt_{0.06}O_{3-\sigma}$.

The X-ray powder diffraction analysis of the powder revealed that it was identified as a single crystal phase comprising a Pt-containing perovskite-type composite oxide having a structure of $Sr_{0.90}Ce_{0.94}Pt_{0.06}O_{3-\sigma}$.

Example 6

(Production of $Sr_{1.00}Ce_{0.47}Zr_{0.47}Pt_{0.06}O_3$)
Strontium isopropoxide     (Sr content: 0.100 mol)
Cerium isopropoxide     (Ce content: 0.047 mol)
Zirconium isopropoxide     (Zr content: 0.047 mol)

The above components were charged in a round-bottomed flask and, after treating in the same manner as in Example 2, an aqueous dinitrodiammine platinum nitrate solution (Pt content: 0.006 mol) was added, followed by stirring at room temperature for one hour.

Next, water was distilled off to dryness under reduced pressure to obtain a precursor of a SrCeZrPt composite oxide. The precursor was subjected to a heat treatment (baking) at 800°C in the atmosphere for 2 hours using an electric furnace to obtain a powder of a Pt-containing perovskite-type composite oxide (Pt content: 4.57% by weight) having a structure of $Sr_{1.00}Ce_{0.41}Zr_{0.47}Pt_{0.06}O_3$.

The X-ray powder diffraction analysis of the powder revealed that it was identified as a single crystal phase comprising a Pt-containing perovskite-type composite oxide having a structure of $Sr_{1.00}Ce_{0.47}Zr_{0.47}Pt_{0.06}O_3$.

Example 7

(Production of $Ca_{1.00}Ce_{0.44}Zr_{0.44}Y_{0.06}Pt_{0.06}O_3$)
Calcium isopropoxide     (Ca content: 0.100 mol)
Cerium isopropoxide     (Ce content: 0.044 mol)

(continued)

(Production of $Ca_{1.00}Ce_{0.44}Zr_{0.44}Y_{0.06}Pt_{0.06}O_3$)
Zirconium isopropoxide    (Zr content: 0.044 mol)
Yttrium isopropoxide      (Y content: 0.006 mol)

An alkoxide mixed solution was prepared by charging the above components in a round-bottomed flask and dissolving them in 200 mL of toluene added thereto with stirring. Deionized water was added to the alkoxide mixed solution to form a viscous precipitate on hydrolysis. Toluene was distilled off from the alkoxide mixed solution to obtain a slurry, and an aqueous dinitrodiammine platinum nitrate solution (Pt content: 0.007 mol) was added to the slurry, followed by stirring at room temperature for one hour.

Next, water was distilled off to dryness under reduced pressure to obtain a precursor of a CaCeZrYPt composite oxide. The precursor was subjected to a heat treatment (baking) at 900°C in the atmosphere for 2 hours using an electric furnace to obtain a powder of a Pt-containing perovskite-type composite oxide (Pt content: 5.66% by weight) having a structure of $Ca_{1.00}Ce_{0.44}Zr_{0.44}Y_{0.06}Pt_{0.06}O_3$.

The X-ray powder diffraction analysis of the powder revealed that it was identified as a single crystal phase comprising a pt-containing composite oxide having a perovskite structure of $Ca_{1.00}Ce_{0.44}Zr_{0.44}Y_{0.06}Pt_{0.06}O_3$.

Comparative Example 1

(Production of $La_{0.95}Sr_{0.05}Al_{0.90}Co_{0.05}Pt_{0.05}O_3$)
Lanthanum ethoxyethylate    (La content: 0.095 mol)
Strontium ethoxyethylate     (Sr content: 0.005 mol)
Aluminum ethoxyethylate     (Al content: 0.090 mol)
Cobalt ethoxyethylate       (Co content: 0.005 mol)

The above components were charged in a round-bottomed flask and, after treating in the same manner as in Example 2, an aqueous dinitrodiammine platinum nitrate solution (Pt content: 0.005 mol) was added, followed by stirring at room temperature for one hour.

Next, water was distilled off to dryness under reduced pressure to obtain a precursor of a LaSrAlCoPt composite oxide. The precursor was subjected to a heat treatment (baking) at 800°C in the atmosphere for 2 hours using an electric furnace to obtain a powder of a Pt-containing perovskite-type composite oxide (Pt content: 4.41% by weight) having a structure of $La_{0.95}Sr_{0.05}Al_{0.90}Co_{0.05}Pt_{0.05}O_3$.

The X-ray powder diffraction analysis of the powder revealed that it was identified as a single crystal phase comprising a Pt-containing perovskite-type composite oxide having a structure of $La_{0.95}Sr_{0.05}Al_{0.90}Co_{0.05}Pt_{0.05}O_3$

Comparative Example 2

(Production of $Pt/\alpha\text{-}Al_2O_3$)

A commercially available $\alpha\text{-}Al_2O_3$ (specific surface area: 13 m$^2$/g) was impregnated with Pt using an aqueous dinitrodiammine platinum nitrate solution, subjected to forced-air drying at 60°C for 24 hours and subjected to a heat treatment at 500°C in the atmosphere for one hour using an electric furnace. The amount of Pt supported on $\alpha\text{-}Al_2O_3$ was 2.00% by weight.

Test Example 1 (Test on physical properties)

1) Measurement of specific surface area

The specific surface areas of the above prepared powders according to Examples and Comparative Examples were measured according to the BET method. The results are shown in Table 1.

2) Measurement of rate of solid solution of noble metal

Each of the powders obtained in Examples and Comparative Examples was subjected to an oxidative treatment (at 800°C in the atmosphere for one hour) and a reducing treatment (a heat treatment at 800°C in a N$_2$ gas containing 10% H$_2$ for one hour), dissolved in an aqueous 7 wt% hydrofluoric acid solution and allowed to stand at room temperature for 20 hours, and then each solution was filtered through a filter having a pore size of 0.1 $\mu$m$\varphi$. The amount of the noble metal dissolved in the filtrate was determined by inductively coupled plasma (ICP) emission spectroscopy and quantitative analysis of the noble metal in the residue was carried out by an XRD (X-ray diffraction) analytical method. A rate of solid solution of the noble metal after the oxidative treatment and the reducing treatment was calculated based on these results. Further, a rate of deposition of the noble metal was calculated from a difference between a rate of a solid solution after an oxidative treatment and a rate of a solid solution after a reducing treatment. The results are shown in Table 1.

In the above method, the residue of a fluoride was produced during dissolving the each powder in an aqueous 7 wt% hydrofluoric acid solution. However, since the noble metal constituting a solid solution in a perovskite-type crystal structure was dissolved, the proportion of the noble metal constituting a solid solution in the perovskite-type crystal structure could be determined by measuring the concentration of the noble metal in the solution.

Test Example 2 (Endurance test)

1) Oxidation-reduction endurance test

One cycle was set as follows: exposure to an inert atmosphere for 5 minutes, exposure to an oxidative atmosphere for 10 minutes, exposure to an inert atmosphere for 5 minutes and exposure to a reducing atmosphere for 10 minutes (30 minutes in total), and this cycle was repeated 20 times for 10 hours in total. Each of the powders obtained in Examples and Comparative Examples was alternately exposed to an oxidative atmosphere and a reducing atmosphere, and then cooled to room temperature while maintaining in the reducing atmosphere.

The inert atmosphere, the oxidative atmosphere or the reducing atmosphere corresponds to an exhaust gas atmosphere in case of burning a mixed air in the stoichiometric state, the lean state or the rich state, respectively.

Each atmosphere was prepared by feeding a gas with the composition shown in Table 2, which contains high temperature steam, at a flow rate of $300 \times 10^{-3}$ m$^3$/hr. The atmospheric temperature was maintained at about 1,000°C.

Table 2

|  | Oxidative atmosphere (vol%) | Inert atmosphere (vol%) | Reducing atmosphere (vol%) |
|---|---|---|---|
| $H_2$ | - | - | 0.5 |
| CO | - | - | 1.5 |
| $O_2$ | 1.0 0 | - | - |
| $CO_2$ | 8.0 | 8.0 | 8.0 |
| $H_2O$ | 10 | 10 | 10 |
| $N_2$ | 81 | 82 | 80 |

2) Evaluation of activity

While a gas (He balance) containing 4% NO and 6% $H_2$ was allowed to flow at a flow rate of 50 mL/min in total, 40 mg of each powder subjected to the oxidation-reduction endurance test was heated from room temperature to 400°C at a rate of 3°C/min. During heating, a signal of NO (mass: 30) was observed by mass spectrometry. The temperature, at which the number of counts decreased by 30% as compared with that as measured at room temperature, was taken as a NO 30% purification temperature. The results are shown in Table 1.

Table 1

| Examples and Comparative Examples | Composition | Content of noble metal (% by weight) | Preparation method | Specific surface area (m²/g) |
|---|---|---|---|---|
| Example 1 | $Ba_{1.00}Ce_{0.93}Pt_{0.07}O_3$ | 4.15 | Alkoxide | 0.6 |
| Example 2 | $Ba_{0.95}Ce_{0.465}Pr_{0.465}Pt_{0.07}O_{3-\sigma}$ | 4.23 | Alkoxide | 0.8 |
| Example 3 | $Ca_{0.80}Ce_{0.95}Pt_{0.05}O_{3-\sigma}$ | 4.38 | Alkoxide | 17.0 |
| Example 4 | $Ca_{1.02}Ce_{0.475}Zr_{0.475}Pt_{0.05}O_{3+\sigma}$ | 4.68 | Alkoxide | 13.0 |
| Example 5 | $Sr_{0.90}Ce_{0.94}Pt_{0.06}O_{3-\sigma}$ | 4.33 | Alkoxide | 0.2 |
| Example 6 | $Sr_{1.00}Ca_{0.47}Zr_{0.47}Pt_{0.06}O_3$ | 4.57 | Alkoxide | 2.0 |
| Example 7 | $Ca_{1.00}Ce_{0.44}Zr_{0.44}Y_{0.06}Pt_{0.06}O_3$ | 5.66 | Alkoxide | 11.0 |
| Comparative Example 1 | $La_{0.95}Sr_{0.05}Al_{0.90}Co_{0.05}Pt_{0.05}O_3$ | 4.41 | Alkoxide | 11.0 |
| Comparative Example 2 | $Pt/\alpha-Al_2O_3$ | 2.00 | Supporting method | 13.0 |

Table 1 (continued)

| Examples and Comparative Examples | Rate of solid solution of noble metal (%) | | Precipitation rate (%) (Oxidation-reduction) | NO 30% purification temperature (°C) |
|---|---|---|---|---|
| | Oxidation | Redution | | |
| Example 1 | 89 | 0 | 89 | 141 |
| Example 2 | 82 | 2 | 80 | 135 |
| Example 3 | 85 | 0 | 85 | 122 |
| Example 4 | 98 | 8 | 90 | 118 |
| Example 5 | 91 | 0 | 91 | 132 |
| Example 6 | 96 | 4 | 92 | 123 |
| Example 7 | 89 | 0 | 89 | 119 |
| Comparative Example 1 | 99 | 71 | 28 | 318 |
| Comparative Example 2 | 0 | 0 | 0 | > 400 |

While the illustrative embodiment of the present invention are provided in the above description, such is for illustrative purpose only and is not to be construed restrictively. Variations of the present invention which will be obvious to those skilled in the art are to be covered in the following claims.

Industrial Applicability

The catalyst composition of the present invention can be preferably used as a reaction catalyst for vapor or liquid phase, for example, an exhaust gas purifying catalyst which is used for purifying exhaust gas exhausted from internal combustion engines such as gasoline engine and diesel engine, and boilers.

Claims

1. A catalyst composition comprising a perovskite-type composite oxide represented by a general formula (1):

$$A_xA'_wB_yB'_{(1-y-z)}N_zO_{3\pm\sigma} \qquad (1)$$

(wherein A represents at least one element selected from alkaline earth metals; A' represents at least one element selected from rare earth elements; B represents a tetravalent rare earth element; B' represents at least one element selected from transition elements (excluding tetravalent rare earth elements, Rh, Pd and Pt); N represents at least one element selected from Rh, Pd and Pt; x and w represent an atomic ratio satisfying the following relations: $0.8 \leq x + w \leq 1.3$ ($0.8 \leq x \leq 1.3$, $0 \leq w \leq 0.4$); y represents an atomic ratio satisfying the following relation: $0 < y < 1.0$; z represents an atomic ratio satisfying the following relation: $0 < z \leq 0.5$; and σ represents an oxygen excess or deficiency amount).

2. The catalyst composition according to claim 1, wherein B is at least one element selected from Ce, Pr and Tb in the general formula (1).

3. The catalyst composition according to claim 1, wherein N represents Pt in the general formula (1).

4. The catalyst composition according to claim 1, wherein at least one element represented by N selected from Rh, Pd and Pt exists as a solid solution at an amount of 50% by weight or more based on each content of at least one element represented by N and selected from Rh, Pd and Pt in a perovskite-type composite oxide under an oxidative atmosphere.

5. The catalyst composition according to claim 1, which is an exhaust gas purifying catalyst.

## INTERNATIONAL SEARCH REPORT

| International application No. |
|---|
| PCT/JP2006/311105 |

A. CLASSIFICATION OF SUBJECT MATTER
*B01J23/58(2006.01)i, B01D53/94(2006.01)i, F01N3/10(2006.01)i*

According to International Patent Classification (IPC) or to both national classification and IPC

B. FIELDS SEARCHED

Minimum documentation searched (classification system followed by classification symbols)
B01J23/58, B01D53/94, F01N3/10

Documentation searched other than minimum documentation to the extent that such documents are included in the fields searched
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CA(STN), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X | JP 2002-204955 A (Toyota Motor Corp.), 23 July, 2002 (23.07.02), Claims; examples 1 to 5 (Family: none) | 1-5 |
| X | JP 10-358 A (Toyota Motor Corp.), 06 January, 1998 (06.01.98), Claim 2; example 5 & US 5906959 A & EP 754494 A2 & DE 69633889 T | 1-5 |

☒ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 August, 2006 (07.08.06) | 22 August, 2006 (22.08.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.

PCT/JP2006/311105

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 2004-41868 A (Daihatsu Motor Co., Ltd.), 12 February, 2004 (12.02.04), Claims & EP 1533031 A1 & EP 1535662 A1 & EP 1535663 A1 & WO 2004/004897 A1 & WO 2004/004898 A1 & WO 2004/004899 A1 & US 2005/0245391 A1 & US 2005/0255993 A1 & CN 1665590 A & CN 1674984 A & CN 1674985 A | 1-5 |

Form PCT/ISA/210 (continuation of second sheet) (April 2005)

**EP 1 894 624 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004041868 A
- JP 2004041867 A
- JP 2004041866 A

**Non-patent literature cited in the description**

- **Y. NISHIHATA et al.** *Nature,* 11 July 2002, vol. 418 (6894), 164-167